# FASCICULE DE BREVET EUROPEEN

(11) **EP 0 649 406 B1**
(45) Date de publication et mention de la délivrance du brevet: **23.12.1998**
(21) Numéro de dépôt: 94915605.3
(22) Date de dépôt: 10.05.1994
(51) Int. Cl.: C07D 209/80, A61K 7/00, C09B 5/26

(54) **DERIVES DE CARBAZOLE ET LEUR UTILISATION EN COSMETIQUE, EN PARTICULIER DANS LE MAQUILLAGE**
CARBAZOLDERIVATE UND IHRE VERWENDUNG IN DER KOSMETIK, INSBESONDERE IN DER SCHMINKE
CARBAZOLE DERIVATIVES AND THEIR USE IN COSMETICS, ESPECIALLY FOR MAKE-UP

(30) Priorité: 10.05.1993 FR 9305588
(43) Date de publication de la demande: 26.04.1995
(73) Titulaire: L'OREAL, 75008 Paris (FR)
(72) Inventeur: HOCQUAUX, Michel, F-75012 Paris (FR); PHILIPPE, Michel, F-92160 Antony (FR)
(74) Mandataire: Casalonga, Axel
(86) Numéro de dépôt international: FR9400549
(87) Numéro de publication internationale: WO9426711

(56) Documents cités:
- EP-A- 0 562 945
- TETRAHEDRON LETTERS, no.15, 1974 pages 1361 - 1362 K.K. PRASAD 'A reinvestigation of the reaction of indole with 1,4-quinones' cité dans la demande

## Description

La présente invention a pour objet de nouveaux dérivés carbazole et leur procédé de préparation, ainsi que leur utilisation en cosmétique, les compositions cosmétiques les contenant et les procédés de traitement cosmétique les mettant en oeuvre.

On est à la recherche dans le domaine cosmétique et en particulier pour les compositions de maquillage telles des compositions de fond de teint, des crèmes teintées, des mascaras, des fards à joues et à paupières, des rouges à lèvres, des vernis à ongles, de pigments susceptibles de donner à ces différents types de produits une palette variée de colorations reproductibles dans le temps, insolubles dans la plupart des milieux cosmétiques utilisés tels que l'eau et les solvants cosmétiquement acceptables. Ces pigments doivent par ailleurs être stables aux pH habituellement utilisés ou rencontrés dans le domaine cosmétique.

On connaît dans l'état de la technique des dérivés de carbazole, produits d'addition de composés indoliques avec des quinones, tels que le produit d'addition de la 1,4-naphtoquinone avec le 1-méthyl 5,6-dihydroxyindole conduisant au 2,3-dihydroxy-5-méthyl-dinaphto[2,3-a:2',3'-c]carbazole-6,11,12,17-tétrone décrit dans les articles de PROTA (Tetrahedron Letters, Vol. 43, n°12 pages 2749-54, 1987 et Gazetta Chemica Italiana, 119, 1989), ou encore le produit d'addition de la 1,4-naphtoquinone avec l'indole conduisant au 5-H-dinaphto[2,3-a:2',3'-c]carbazole-6,11,12,17-tétrone décrit dans l'article de PROTA (Gazetta Chemica Italiana, 119, 1989) et dans l'article de PRASAD (Tetrahedron letters, No. 15, pages 1361-1362, 1974).

La demanderesse vient de découvrir de nouveaux dérivés de carbazole dont l'utilisation à titre de pigments est particulièrement intéressante dans le domaine de la cosmétique et en particulier leur utilisation dans les compositions de maquillage notamment les fonds de teint, crèmes teintées, mascaras, fards à joues et à paupières, rouges à lèvres, vernis à ongles et coloration temporaire des cheveux. Ces pigments permettent d'obtenir une palette très variée de colorations très recherchée dans le domaine cosmétique et reproductible, allant du jaune au marron en passant par le jaune-orangé, l'orange et le rouge vif. Ils sont en outre stables à des pH compris entre 6 et 8 et insolubles dans la plupart des milieux cosmétiques.

En outre la cristallinité élevée de ces pigments empêche le phénomène de relargage des couleurs dans les formulations.

Un objet de l'invention est constitué par de nouveau dérivés de carbazole et leur synthèse.

L'invention a également pour objet l'utilisation de ces pigments dans des compositions cosmétiques.

Un autre objet de l'invention est constitué par les compositions cosmétiques contenant ce pigment, ainsi que leur application pour le traitement cosmétique et/ou la coloration des matières kératiniques telles que la peau, les ongles les cils, les sourcils et les cheveux.

D'autres objets de l'invention apparaîtront à la lecture de la description et des exemples qui suivent.

Les composés nouveaux conformes à la présente invention répondent à la formule (I) 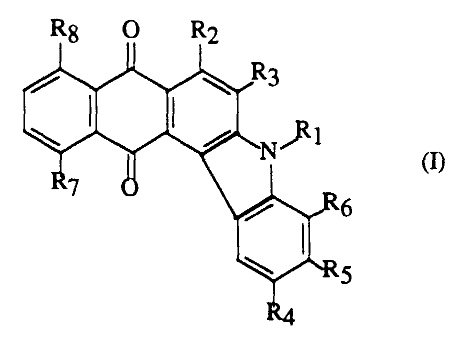 dans laquelle,
R₁ représente un atome d'hydrogène, un radical benzyle, alkyle en C₁-C₁₆ ramifié ou non, saturé ou non;
R₂ et R₃ désignent indépendamment l'un de l'autre le radical carbéthoxy, acétyle, nitrile, 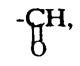 un atome d'hydrogène,
R₂ et R₃ ne pouvant toutefois représenter simultanément un atome d'hydrogène, ou R₂ et R₃ forment les cycles suivants: 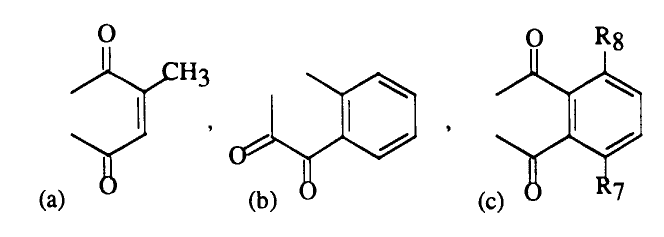
R₄ représente un atome d'hydrogène, le radical méthoxy, nitrile, nitro ou un atome de chlore;
R₅, R₇ et R₈ représentent, indépendamment l'un de l'autre, un atome d'hydrogène ou le radical méthoxy;
R₆ représente un atome d'hydrogène ou le radical méthyle;
sous réserve que si R₂ et R₃ forment le cycle (c), au moins l'un des R₁, R₄, R₅, R₆, R₇ et R₈ ne désigne pas un atome d'hydrogène.

Les composés particulièrement préférés sont choisis parmi :
- la 2,3-diméthoxy-5-H-dinaphto[2,3-a: 2',3'-c] carbazole-6,11,12,17-tétrone,
- la 10,13-diméthoxy-5H-dinaphto[2,3-a: 2',3'-c]carbazole 6,11,12,17-tétrone,
- la 7,16-diméthoxy-5H-dinaphto[2,3- a: 2',3'-c]carbazole-6,11,12,17-tétrone,
- la 7,13-diméthoxy-5H-dinaphto[2,3-a: 2',3'-c]carbazole-6,11,12,17-tétrone,
- la 10,16-diméthoxy-5H-dinaphto[2,3-a: 2',3'-c]carbazole-6,11,12,17 tétrone,
- la 2-méthoxy-5H-dinaphto[2,3-a : 2',3'-c]carbazole-6,11,12,17-tétrone,
- la 4-méthyl-5H-dinaphto[2,3-a: 2',3'-c]carbazole-6,11,12,17-tétrone,
- la 2-chloro-5H-dinaphto[2,3-a: 2',3'-c]carbazole-6,11,12,17-tétrone,
- la 7-méthyl-5-hexadécyl-naphto[2,3-a]-benzo[2',3'-c]carbazole-6,9, 10,15-tétrone,
- la 5-hexadécyl-dinaphto[2,3-a: 2',3'-c]carbazole-6, 11,12,17-tétrone,
- la 2-cyano-5H-dinaphto[2,3-a: 2',3'-c]carbazole-6,11,12,17-tétrone,
- la 2-nitro-10,13-diméthoxy 5H-dinaphto[2,3-a: 2',3'-c]carbazole-6,11,12,17-tétrone,
- la 2-nitro-7,16-diméthoxy 5H-dinaphto[2,3-a: 2',3'-c]carbazole-6,11, 12,17-tétrone,
- la 2-nitro-7,13-diméthoxy 5H-dinaphto[2,3-a: 2',3'-c]carbazole-6,11, 12,17-tétrone,
- la 2-nitro-10,16-diméthoxy 5H-dinaphto[2,3-a: 2',3'-c]carbazole-6, 11,12,17-tétrone,

Les composés de formule (I) conformes à la présente invention peuvent être obtenus par deux procédés de préparation différents.

1) On peut préparer les composés de formule (I) dans laquelle R₂ et R₃ forment le cycle (c) de la façon suivante :
on fait réagir en milieu acide une 1,4-naphtoquinone de formule (II) 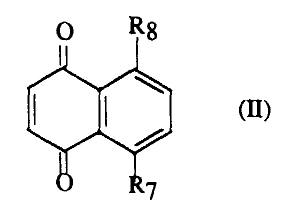 dans laquelle R₇ et R₈ représentent indépendamment l'un de l'autre soit un atome d'hydrogène soit le radical méthoxy,
avec un composé indolique de formule (III) 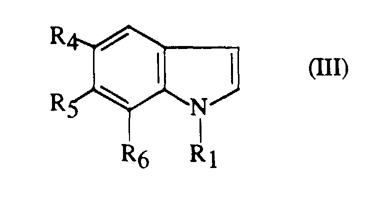 dans laquelle
R₁ représente un atome d'hydrogène, un radical benzyle ou alkyle en C₁-C₁₆ ramifié ou non, saturé ou non;
R₄ représente un atome d'hydrogène, le radical méthoxy, nitrile ou nitro ou un atome de chlore;
R₅ désigne un atome d'hydrogène ou le radical méthoxy,
R₆ désigne un atome d'hydrogène ou le radical méthyle,
sous réserve que si R₇ et R₈ désignent chacun un atome d'hydrogène,
au moins l'un des R₁, R₄, R₅ et R₆, soit différent d'un atome d'hydrogène.

L'addition de la 1,4-naphtoquinone en position 2 ou 3 du composé indole est suivie d'une cyclisation pour obtenir le dérivé cabazole de formule (I) dans laquelle R₂ et R₃ forment le cycle (c).

Le milieu réactionnel acide est constitué de préférence par de l'acide acétique glacial ou de l'acide acétique aqueux. On opère généralement à température ambiante ou au reflux de l'acide acétique.

2) Les composés de formule (I) peuvent être préparés par un deuxième procédé.

a/ Dans un premier temps, on fait réagir dans un milieu acide une 1,4-naphtoquinone de formule (II) : 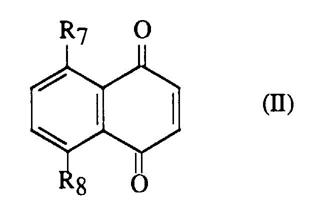 dans laquelle, R₇ et R₈, indépendamment l'un de l'autre représentent un atome d'hydrogène ou le radical méthoxy,
avec un composé indolique de formule (III): 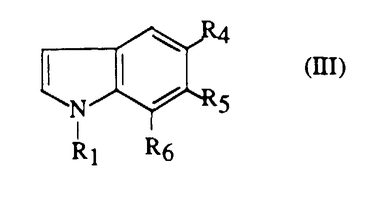 dans laquelle,
R₁ représente un atome d'hydrogène, un radical benzyle, alkyle en C₁-C₁₆ ramifié ou non, saturé ou non;
R₄ représente un atome d'hydrogène, le radical nitrile, méthoxy, nitro ou un atome de chlore;
R₅ représente un atome d'hydrogène ou le radical méthoxy;
R₆ représente un atome d'hydrogène ou le radical méthyle;
le milieu réactionnel utilisé est de préférence constitué par de l'acide acétique glacial, de l'acide acétique aqueux, de l'éthanol avec quelques gouttes d'acide chlorhydrique, selon BULOK & MASON (J. Chem. Soc. 1951, 703). On opère généralement à température ambiante ou au reflux de l'acide acétique.

On obtient le composé indolyl-1,4-naphtoquinone de formule (IV) : 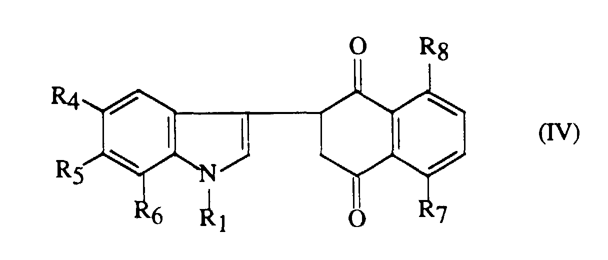 dans laquelle R₁, R₄, R₆, R₇ et R₈ ont les mêmes significations indiquées ci-dessus.

b/ On fait réagir dans un milieu réactionnel acide constitué de préférence par de l'acide acétique glacial ou de l'acide acétique aqueux, ce composé indolyl-1,4-naphtoquinone de formule (IV) avec un diénophile choisi parmi : 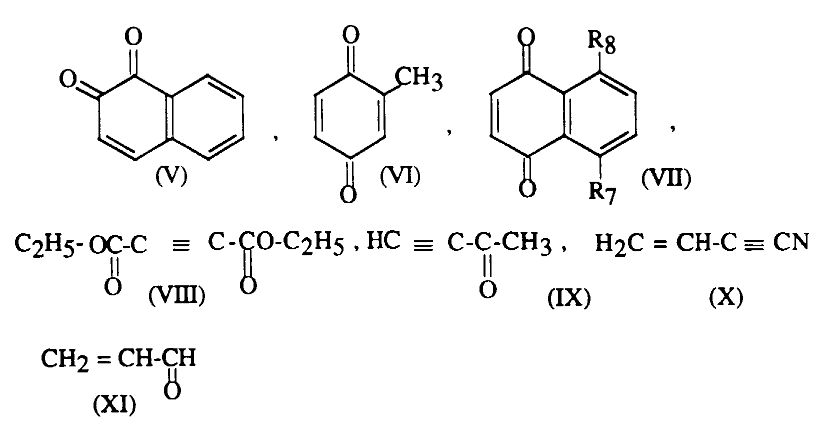 avec R₇ et R₈ ayant les mêmes significations indiquées ci-dessus; sous réserve que si l'on fait réagir le composé indolyl-1,4-naphtoquinone de formule (IV) avec le diénophile de formule (VII), R₁, R₄, R₅, R₆, R₇ et R₈ ne peuvent désigner simultanément un atome d'hydrogène.

Un autre objet de l'invention est l'utilisation de ce composé de formule (I), à titre de pigment dans des compositions cosmétiques pour le traitement et/ou la coloration des matières kératiniques telles que la peau, les ongles et les cheveux. Etant donné son caractère insoluble, ce pigment est utilisé plus particulièrement dans les compositions de fond de teint, des crèmes teintées, des mascaras, des fards à joues et à paupières, des rouges à lèvres, des vernis à ongles et des compositions de coloration temporaire des cheveux. Il peut être utilisé seul ou en mélange avec d'autres pigments minéraux ou organiques classiques.

Les compositions cosmétiques qui constituent un autre objet de l'invention sont caractérisées par le fait qu'elles contiennent au moins un composé répondant à la formule (I) définie ci-dessus dans un milieu cosmétiquement acceptable.

Le pigment de formule (I) est utilisé généralement à une concentration pouvant varier entre 0,1 et 80% en poids et de préférence entre 1 et 30% en poids par rapport au poids total de la composition.

Le milieu cosmétiquement acceptable est un milieu essentiellement non solvant du pigment.

On appelle milieu essentiellement non solvant un milieu qui dissout moins de 1% dudit pigment.

Les compositions peuvent se présenter sous forme de lotion, de lotion épaissie, de gel, d'émulsions (crèmes, laits, pommades), de dispersions vésiculaires, de poudre, de stick. Elles peuvent éventuellement être conditionnées en aérosol et se présenter sous forme de mousse ou de spray.

Des compositions sont de préférence des émulsions de type huile-dans-eau ou eau-dans-huile ou des dispersions liposomales ou encore des préparations solides.

Lorsqu'elles sont utilisées sous forme d'émulsions, elles peuvent contenir en outre des agents tensio-actifs bien connus dans l'état de la technique tels que des agents tensio-actifs anioniques, non ioniques, cationiques, amphotères ou leurs mélanges.

Ces compositions peuvent également contenir des corps gras, des solvants organiques, des silicones, des épaississants, des adoucissants, des filtres solaires, des agents anti-radicaux libres, des agents anti-mousses, des agents hydratants, des parfums, des conservateurs, des agents anti-oxydants, des charges, des séquestrants, des agents de traitement tels que des polymères non-ioniques cationiques, anioniques, amphotères ou leurs mélanges, des propulseurs, des agents alcalinisants ou acidifiants.

Les corps gras peuvent être constitués par une huile ou une cire ou leurs mélanges, les acides gras, les alcools gras, les esters d'acides gras, la vaseline, la paraffine, la lanoline, la lanoline hydrogénée, la lanoline acétylée.

Les huiles sont choisies parmi les huiles animales, végétales, minérales, de synthèse et notamment l'huile de palme hydrogénée, l'huile de ricin hydrogénée, l'huile de vaseline, l'huile de paraffine, l'huile de purcellin, les huiles de silicone.

Les cires sont choisies parmi les cires animales, fossiles, végétales, minérales ou de synthèse. On peut citer plus particulièrement, les cires d'abeilles, les cires de carnauba, de candelilla, de canne à sucre, du Japon, les ozokérites, la cire de montan, les cires microcristallines, les paraffines.

Lorsque les compositions sont utilisées pour la coloration des ongles, elles se présentent sous forme de produits dits "vernis à ongles" contenant le pigment conforme à l'invention sous forme dispersée dans un solvant cosmétiquement acceptable contenant une ou plusieurs résines et des ingrédients habituellement utilisés dans ce type de produits.

Lorsque les compositions sont utilisées pour la coloration des cheveux, elles comprennent de préférence un milieu approprié pour la coloration temporaire constitué par de l'eau, un solvant ou un mélange d'eau et d'un ou plusieurs solvant(s) cosmétiquement acceptable(s), contenant au moins un pigment conforme à l'invention sous forme dispersée.

Les solvants sont choisis plus particulièrement parmi les alcools inférieurs en C₁-C₆, les alkylèneglycols comme l'éthylèneglycol ou le propylèneglycol, les éthers de glycol comme les éthers monométhylique, monoéthylique ou monobutylique de l'éthylèneglycol, l'acétate de monoéthyléther de l'éthylèneglycol, les monométhyléthers du propylèneglycol et du dipropylèneglycol, le lactate de méthyle.

Les solvants particulièrement préférés sont l'alcool éthylique et le propylèneglycol.

Les compositions conformes à l'invention peuvent également contenir en plus du pigment défini ci-dessus, d'autres pigments généralement utilisés en cosmétique notamment des pigments blancs ou colorés, des pigments nacrés et/ou nacrants permettant de faire varier les colorations ou encore d'augmenter la protection vis-à-vis du rayonnement ultraviolet. Parmi ces pigments on peut utiliser des nanopigments d'oxydes métalliques, tels que les oxydes de titane, de zinc, de cérium et/ou de zirconium ayant un diamètre moyen inférieur à 100 nm de préférence compris entre 1 et 50 nm. Ces pigments peuvent être enrobés.

Un autre objet de l'invention est constitué par un procédé de coloration ou de maquillage des matières kératiniques telles que la peau, les ongles, les cils, les sourcils et les cheveux, mettant en oeuvre une composition contenant dans une milieu cosmétiquement acceptable au moins un pigment répondant à la formule (I) telle que définie ci-dessus.

Les exemples suivants sont destinés à illustrer l'invention sans pour autant présenter un caractère limitatif.

### Exemples de préparation

Les composés de formule (I) peuvent être obtenus par deux procédés de préparation.

### Procédé de préparation A

Dans un tricol muni d'un thermomètre, d'un réfrigérant et d'une agitation magnétique, on agite à une température comprise entre 45 et 90°C un mélange de 6,5 mmoles de 1,4-naphtoquinone éventuellement substituée répondant à la formule (II), de 3 mmoles d'un composé indolique de formule (III) et de 10 à 30 ml d'acide acétique pendant 6 à 48 heures. On laisse ensuite revenir à température ambiante.

On filtre le mélange réactionnel, puis on lave le précipité obtenu avec de l'eau, de l'éthanol et éventuellement de l'acétone.

Les rendements sont compris entre 15 et 50%.

### Procédé de préparation B

Dans un tricol muni d'un thermomètre, d'un réfrigérant et d'une agitation magnétique, on agite à une température comprise entre 45 et 90°C un mélange de 5 mmoles de 1,4-monoindolylnaphtoquinone de formule (IV), de 3 mmoles d'un diénophile répondant à l'une des formules (V) à (XI) et de 15 à 30 ml d'acide acétique pendant 6 à 48 heures. On laisse ensuite revenir à température ambiante.

On filtre le mélange réactionnel, puis on lave le précipité obtenu avec de l'eau, de l'éthanol et éventuellement de l'acétone.

Eventuellement on effectue une chromatographie sur colonne de gel de silice.

Les rendements sont compris entre 10 et 50%.

### Exemple 1

### Préparation du 2,3-diméthoxy-5H-dinaphto[2,3-a: 2',3'-c]carbazole-6,11,12,17-tétrone

Ce composé est préparé suivant le procédé de préparation A. On obtient un produit de couleur marron présentant les caractéristiques suivantes :
Point de fusion: > à 350°C

| Analyse élémentaire : | | | | |
|---|---|---|---|---|
| | C% | H% | N% | O% |
| Théorie | 73,85 | 3,49 | 2,87 | 19,69 |
| Trouvé | 73,41 | 3,99 | 2,66 | 19,75 |

### Exemple 2

### Préparation du mélange : 10,13-diméthoxy-5H-dinaphto[2,3-a: 2',3'-c]carbazole-6,11,12,17-tétrone, 7,16-diméthoxy-5H-dinaphto[2,3-a: 2',3'-c]carbazole-6,11,12,17-tétrone, 7,13-diméthoxy-5H-dinaphto[2,3-a: 2',3'-c]carbazole-6,11,12,17-tétrone, 10,16-diméthoxy-5H-dinaphto[2,3-a: 2',3'-c]carbazole-6,11,12,17-tétrone,

Ce mélange est obtenu par la mise en oeuvre du procédé de préparation A. On obtient un produit de couleur orange qui présente les caractéristiques suivantes :
Point de fusion : > à 260°C

| Analyse élémentaire : | | | | |
|---|---|---|---|---|
| | C% | H% | N% | O% |
| Théorie | 73,85 | 3,49 | 2,87 | 19,69 |
| Trouvé | 73,51 | 3,90 | 2,68 | 19,52 |

### Exemple 3

### Préparation du 2-méthoxy-5H-dinaphto[2,3-a: 2',3'-c]carbazole-6,11,12,17-tétrone.

Ce composé est obtenu par la mise en oeuvre du procédé de préparation A. On obtient un produit marron présentant les caractéristiques suivantes :
Point de fusion : > à 260°C

| Analyse élémentaire : | | | | |
|---|---|---|---|---|
| | C% | H% | N% | O% |
| Théorie | 76,15 | 3,31 | 3,06 | 17,49 |
| Trouvé | 75,94 | 3,28 | 2,90 | 17,39 |

### Exemple 4

### Préparation du 4-méthyl-5H-dinaphto[2,3-a: 2',3'-c]carbazole-6,11,12,17-tétrone.

Ce composé est obtenu en mettant en oeuvre le procédé de préparation A. On obtient un produit de couleur rouge présentant les caractéristiques suivantes :
Point de fusion : > à 260°C

| Analyse élémentaire : | | | | |
|---|---|---|---|---|
| | C% | H% | N% | O% |
| Théorie | 78,90 | 3,42 | 3,17 | 14,50 |
| Trouvé | 79,15 | 3,36 | 2,98 | 14,34 |

### Exemple 5

### Préparation du 2-chloro-5H-dinaphto[2,3-a: 2',3'-c]carbazole-6,11,12,17-tétrone.

Ce composé est obtenu par la mise en oeuvre du procédé de préparation A. On obtient un produit de couleur orange présentant les caractéristiques suivantes :
Point de fusion : > à 260°C

| Analyse élémentaire : | | | | |
|---|---|---|---|---|
| | C% | H% | N% | O% |
| Théorie | 72,82 | 2,62 | 3,03 | 13,86 |
| Trouvé | 72,30 | 2,63 | 2,91 | 13,94 |

### Exemple 6

### Préparation du 7-méthyl-5-hexadécyl-naphto[2,3-a]-benzo[2'-3'-c]carbazole-6,9,10,15-tétrone.

Ce composé est obtenu par la mise en oeuvre du procédé de préparation B. Après chromatographie sur gel de silice en utilisant comme éluant le chloroforme, on obtient un produit de couleur rouge cristallisé avec 0,5 H₂O, présentant les caractéristiques suivantes :
Point de fusion : 130-132°C

| Analyse élémentaire : | | | | |
|---|---|---|---|---|
| | C% | H% | N% | O% |
| Théorie | 78,79 | 7,44 | 2,24 | 11,52 |
| Trouvé | 78,13 | 7,25 | 2,35 | 10,70 |

### Exemple 7

### Préparation du 5-hexadécyl-dinaphto[2,3-a: 2'-3'-c]carbazole-6,11,12,17-tétrone.

Ce composé est obtenu par la mise en oeuvre du procédé de préparation A. On obtient un produit de couleur orange cristallisé avec 0,5 H₂0 présentant les caractéristiques suivantes:
Point de fusion : 124-125°C

| Analyse élémentaire : | | | | |
|---|---|---|---|---|
| | C% | H% | N% | O% |
| Théorie | 81,07 | 6,96 | 2,15 | 9,82 |
| Trouvé | 80,77 | 7,00 | 2,24 | 9,93 |

### Exemple 8

### Préparation du 2-cyano-5H-dinaphto[2,3-a: 2'-3'-c]carbazole-6,11,12,17-tétrone.

Ce composé est obtenu par la mise en oeuvre du procédé de préparation A. On obtient un produit de couleur marron cristallisé avec 0,33 H₂O présentant les caractéristiques suivantes :
Point de fusion : > à 260°C

| Analyse élémentaire : | | | | |
|---|---|---|---|---|
| | C% | H% | N% | O% |
| Théorie | 75,91 | 2,76 | 6,10 | 15,11 |
| Trouvé | 75,97 | 2,64 | 6,04 | 15,44 |

### Exemple 9

### Préparation du mélange de 2-nitro-10,13-diméthoxy 5H-dinaphto[2,3-a: 2',3'c]carbazole-6, 11, 12,17-tétrone, 2-nitro-7,16-diméthoxy 5H-dinaphto[2,3-a: 2',3'-c]carbazole-6,11, 12,17-tétrone, 2-nitro 7,13-diméthoxy 5H-dinaphto[2,3-a: 2',3'-c]carbazole-6,11, 12,17-tétrone, et 2-nitro 10,16-diméthoxy 5H-dinaphto[2,3-a: 2',3'-c]carbazole-6,11, 12,17-tétrone,

Ce mélange est obtenu par la mise en oeuvre du procédé de préparation A. On obtient un produit de couleur orange cristallisé avec 1 mole d'eau présentant les caractéristiques suivantes :
Point de fusion : > à 260°C

| Analyse élémentaire : | | | | |
|---|---|---|---|---|
| | C% | H% | N% | O% |
| Théorie | 65,45 | 3,29 | 5,08 | 26,15 |
| Trouvé | 64,75 | 3,29 | 5,40 | 25,02 |

### Exemples de formulations

Poudre pour le visage :
- Talc qsp 100 g
- Poudre de polyéthylène 5,00 g
- Carbonate de magnésium 11,00 g
- Myristate d'isopropyle 1,50 g
- Huile de vaseline 1,50 g
- Sorbitol 0,50 g
- Mélange de composés de l'exemple 9 0,25 g
- Pigment insoluble constitué d'un colorant de la famille des indigos déposé sur charge dénommé DC Red 30 LAKE dans le dictionnaire CTFA 0,65 g
- Ultramarine blue 0,15 g
- Micatitane 5,00 g

Rouge à lèvre
- Huile de ricin qsp 100 g
- Butyl Hydroxy Toluène (BHT) 0,15 g
- Lanoline liquide 15,00 g
- Cire microcristalline 15,00 g
- Huile de sésame 11,00 g
- 1-[[4-(phénylazo)phényl]azo]2-naphtalénol dénommé DC Red n° 17 dans le dictionnaire CTFA 1,00 g
- Composé de l'exemple 8 1,00 g
- Composé de l'exemple 5 2,00 g
- Micatitane 5,00 g
- Béhénate d'octyl glycérol 15,00 g
- Parfum qs

Vernis à ongle
- Toluène qsp 100 g
- Nitrocellulose 10,90 g
- Résine toluène sulfonamide formaldéhyde 9,85 g
- Acétyltributyl citrate 6,50 g
- Acétate de butyle 21,80 g
- Acétate d'éthyle 9,40 g
- Stéaral konium hectorite 1,36 g
- Ultramarine blue 0,01 g
- Dioxyde de titane 0,77 g
- Sel de calcium de l'acide 3-hydroxy 4-[(1-sulfo 2-naphtalényl)azo]2-naphtalène carboxylique dénommé DC Red 34 dans le dictionnaire CTFA 0,07 g
- Composé de l'exemple 5 0,50 g
- Micatitane 0,60 g
- Alcool isopropylique 7,80 g

## Revendications

1. Composé répondant à la formule : 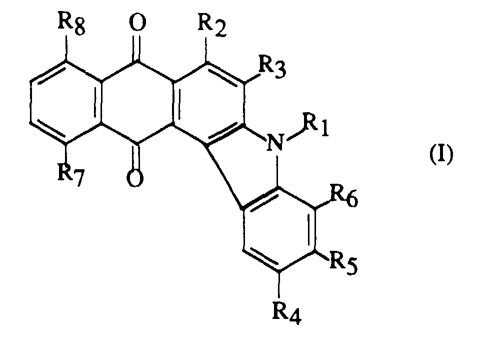 dans laquelle,
R₁ représente un atome d'hydrogène, un radical benzyle, alkyle en C₁-C₁₆ ramifié ou non, saturé ou non;
R₂ et R₃ désignent indépendamment l'un de l'autre le radical carbéthoxy, acétyle, nitrile, 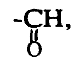 un atome d'hydrogène,
R₂ et R₃ ne pouvant toutefois représenter simultanément un atome d'hydrogène, ou R₂ et R₃ forment les cycles suivants : 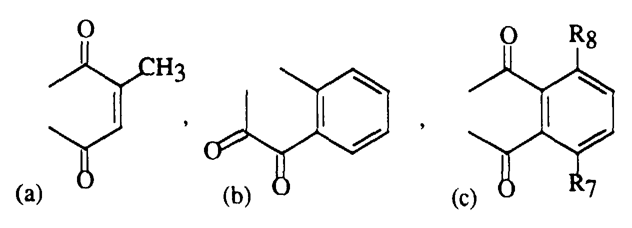
R₄ représente un atome d'hydrogène, le radical méthoxy, nitrile, nitro ou un atome de chlore;
R₅, R₇ et R₈ représentent, indépendamment l'un de l'autre, un atome d'hydrogène ou le radical méthoxy;
R₆ représente un atome d'hydrogène ou le radical méthyle;
sous réserve que si R₂ et R₃ forment le cycle (c), au moins l'un des R₁, R₄, R₅ R₆ R₇ et R₈ ne désigne pas un atome d'hydrogène.

2. Composé selon la revendication 1, caractérisé en ce qu'il est choisi parmi:
- la 2,3-diméthoxy-5-H-dinaphto[2,3-a: 2',3'-c] carbazole-6,11,12,17-tétrone,
- la 10,13-diméthoxy-5H-dinaphto[2,3-a: 2',3'-c]carbazole 6,11,12,17-tétrone,
- la 7,16-diméthoxy-5H-dinaphto[2,3- a : 2',3'-c]carbazole-6,11,12,17-tétrone,
- la 7,13-diméthoxy-5H-dinaphto[2,3-a: 2',3'-c]carbazole-6,11,12,17-tétrone,
- la 10,16-diméthoxy-5H-dinaphto[2,3-a: 2',3'-c]carbazole-6,11,12,17 tétrone,
- la 2-méthoxy-5H-dinaphto[2,3-a: 2',3'-c]carbazole-6,11,12,17-tétrone
- la 4-méthyl-5H-dinaphto[2,3-a: 2',3'-c]carbazole-6,11,12,17-tétrone,
- la 2-chloro-5H-dinaphto[2,3-a: 2',3'-c]carbazole-6,11,12,17-tétrone,
- la 7-méthyl-5-hexadécyl-naphto[2,3-a]-benzo[2',3'-c]carbazole-6,9,10,15-tétrone.
- la 5-hexadécyl-dinaphto[2,3-a : 2',3'-c]carbazole-6, 11,12,17-tétrone,
- la 2-cyano-5H-dinaphto[2,3-a: 2',3'-c]carbazole-6,11,12,17-tétrone,
- la 2-nitro-10,13-diméthoxy 5H-dinaphto[2,3-a: 2',3'c]carbazole-6,11,12,17-tétrone,
- la 2-nitro-7,16-diméthoxy 5H-dinaphto[2,3-a: 2',3'-c]carbazole-6,11, 12,17-tétrone,
- la 2-nitro-7,13-diméthoxy 5H-dinaphto[2,3-a: 2',3'-c]carbazole-6,11, 12,17-tétrone,
- la 2-nitro-10,16-diméthoxy 5H-dinaphto[2,3-a: 2',3'-c]carbazole-6, 11,12,17-tétrone.

3. Procédé de préparation des composé de formule (I) selon la revendication 1, caractérisé en ce qu'il consiste à faire réagir, dans un milieu constitué par de l'acide acétique glacial ou de l'acide acétique aqueux à une température variant de la température ambiante à celle du reflux du mélange, une 1,4-naphtoquinone de formule (II) 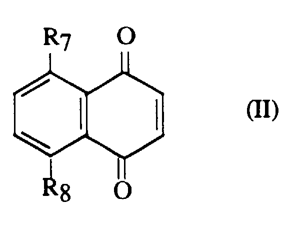 dans laquelle, R₇ et R₈, indépendamment l'un de l'autre représentent un atome d'hydrogène ou le radical méthoxy, avec un composé indolique de formule (III): 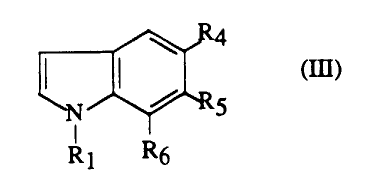 dans laquelle,
R₁ représente un atome d'hydrogène, un radical benzyle, alkyle en C₁-C₁₆ ramifié ou non, saturé ou non;
R₄ représente un atome d'hydrogène, le radical nitrile, méthoxy, nitro ou un atome de chlore;
R₅ représente un atome d'hydrogène ou le radical méthoxy;
R₆ représente un atome d'hydrogène ou le radical méthyle,
à faire réagir dans un même milieu le composé indolyl-1,4-naphtoquinone de formule (IV) ainsi obtenu: 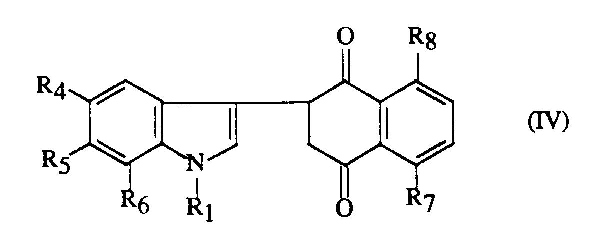 dans laquelle R₁, R₄, R₅, R₆, R₇ et R₈ ont les mêmes significations indiquées plus haut, avec un diénophile choisi parmi ceux répondant aux formules suivantes : 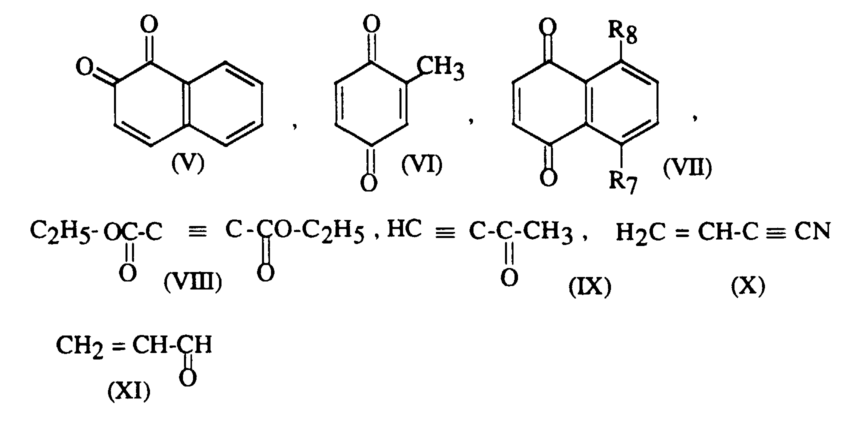 avec R₇ et R₈ ayant les mêmes significations indiquées ci-dessus, sous réserve que si l'on fait réagir l'indolyl-1,4-naphtoquinone de formule (IV) avec le diénophile de formule (VII), R₁, R₄, R₅, R₆, R₇ et R₈ ne peuvent désigner simultanément un atome d'hydrogène.

4. Utilisation à titre de pigment dans des compositions cosmétiques du composé répondant à la formule (I) telle que définie dans la revendication 1.

5. Utilisation selon la revendication 4, caractérisée par le fait que les compositions cosmétiques sont constituées par des compositions de fonds de teint, de crèmes teintées, des mascaras, des fards à joues ou à paupières, des rouges à lèvres, des vernis à ongles et des compositions de coloration temporaire des cheveux.

6. Composition cosmétique destinée au traitement cosmétique ou à la coloration des matières kératiniques, caractérisée par le fait qu'elle contient dans un milieu cosmétiquement acceptable au moins un pigment constitué par le composé répondant à la formule (I) telle que définie dans la revendication 1.

7. Composition selon la revendication 6, caractérisée par le fait que le pigment de formule (I) est présent en une concentration comprise entre 0,1 et 80% et de préférence entre 1 et 30% en poids par rapport au poids total de la composition.

8. Composition selon l'une quelconque des revendications 6 et 7, caractérisée par le fait que la composition se présente sous forme de lotion, de lotion épaissie, de gel, d'émulsion, de dispersion vésiculaire, de poudre, de stick ou est éventuellement conditionnée en aérosol sous forme de spray ou de mousse.

9. Composition selon l'une quelconque des revendications 6 à 8, caractérisée par le fait qu'elle contient des corps gras, des solvants organiques, des silicones, des épaississants, des adoucissants, des tensio-actifs, des filtres solaires, des agents anti-radicaux libres, des agents anti-mousses, des agents hydratants, des parfums, des conservateurs, des agents anti-oxydants, des charges, des séquestrants, des agents de traitement, des propulseurs, des agents alcalinisants ou acidifiants ou d'autres pigments.

10. Composition selon la revendication 6, caractérisée par le fait qu'elle est destinée à être utilisée pour la coloration des ongles et qu'elle se présente sous forme d'une dispersion du pigment dans un solvant cosmétiquement acceptable contenant une ou plusieurs résines.

11. Composition selon la revendication 6, caractérisée par le fait qu'elle est destinée à être utilisée pour la coloration temporaire des cheveux et qu'elle se présente sous forme d'une dispersion du pigment dans un milieu approprié pour la coloration.

12. Procédé de maquillage de la peau, des cils ou des sourcils caractérisé par le fait que l'on applique sur la peau, les cils ou les sourcils au moins une composition telle que définie à l'une quelconque des revendications 6 à 9 et contenant à titre de pigment au moins un pigment de formule (I) telle que définie dans les revendications 1 ou 2.

13. Procédé de coloration des ongles caractérisé par le fait que l'on applique sur les ongles au moins une composition contenant dans un solvant cosmétiquement acceptable, une ou plusieurs résines et au moins un pigment constitué par le composé de formule (I) telle que définie dans les revendications 1 ou 2.

14. Procédé de coloration des fibres kératiniques, en particulier des cheveux, caractérisé par le fait que l'on applique sur les fibres au moins une composition contenant dans un milieu approprié pour la coloration au moins un pigment constitué par le composé de formule (I) telle que définie dans les revendications 1 ou 2.

## Patentansprüche

1. Verbindung der Formel: 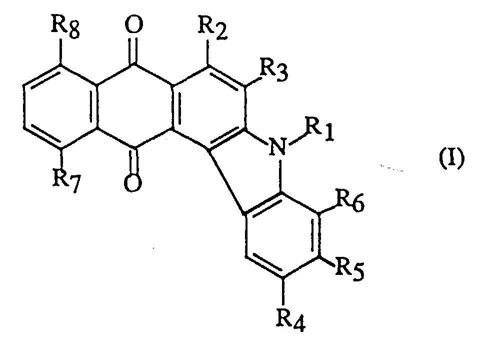 worin gilt:
R₁ stellt ein Wasserstoffatom, einen Benzyl- oder C₁₋₁₆-Alkylrest, verzweigt oder nicht, gesättigt oder nicht, dar;
R₂ und R₃ bedeuten, unabhängig voneinander, den Carbethoxy, Acetyl-, Nitrilrest, 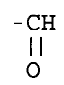 und ein Wasserstoffatom,
wobei R₂ und R₃ nicht gleichzeitig ein Wasserstoffatom darstellen, oder R₂ und R₃ bilden die folgenden Ringe: 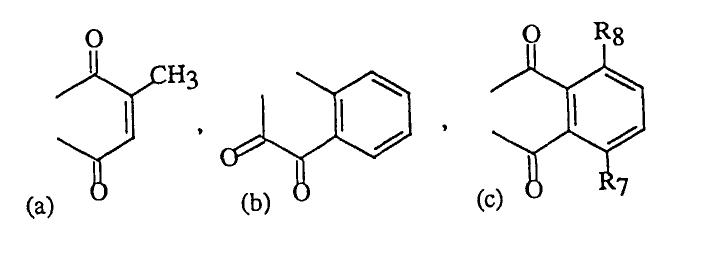
R₄ stellt ein Wasserstoffatom, den Methoxy-, Nitril-, Nitrorest oder ein Chloratom dar;
R₅, R₇ und R₈ stellen, unabhängig voneinander, ein Wasserstoffatom oder den Methoxyrest dar;
R₆ stellt ein Wasserstoffatom oder den Methylrest dar;
mit der Maßgabe, daß, wenn R₂ und R₃ den Ring (c) bilden, mindestens einer der Reste R₁, R₄, R₅, R₆, R₇ und R₈ kein Wasserstoffatom bedeutet.

2. Verbindung gemäß Anspruch 1,
dadurch **gekennzeichnet,** daß
sie ausgewhält ist aus:
2,3-Dimethoxy-5H-dinaphtho[2,3-a:2',3'-c]carbazol-6,11,12,17-tetron,
10,13-Dimethoxy-5H-dinaphtho[2,3-a:2',3'-c]carbazol-6,11,12,17-tetron,
7,16-Dimethoxy-5H-dinaphtho[2,3-a:2',3'-c]carbazol-6,11,12,17-tetron,
7,13-Dimethoxy-5H-dinaphtho[2,3-a:2',3'-c]carbazol-6,11,12,17-tetron,
10,16-Dimethoxy-5H-dinaphtho[2,3-a:2',3'-c]carbazol-6,11,12,17-tetron,
2-Methoxy-5H-dinaphtho[2,3-a:2',3'-c]carbazol-6,11,12,17-tetron,
4-Methyl-5H-dinaphtho[2,3-a:2',3'-c]carbazol-6,11,12,17-tetron,
2-Chlor-5H-dinaphtho[2,3-a:2',3'-c]carbazol-6,11,12,17-tetron,
7-Methyl-5-hexadecylnaphtho[2,3-a]benzo[2',3'-c]carbazol-6,9,10,15-tetron,
5-Hexadecyldinaphtho[2,3-a:2',3'-c]carbazol-6,11,12,17-tetron,
2-Cyano-5H-dinaphtho[2,3-a:2',3'-c]carbazol-6,11,12,17-tetron,
2-Nitro-10,13-dimethoxy-5-H-dinaphtho[2,3-a:2',3'-c]carbazol-6,11,12,17-tetron,
2-Nitro-7,16-dimethoxy-5H-dinaphtho[2,3-a:2',3'-c]carbazol-6,11,12,17-tetron,
2-Nitro-7,13-dimethoxy-5H-dinaphtho[2,3-a:2',3'-c]carbazol-6,11,12,17-tetron und aus
2-Nitro-10,16-dimethoxy-5H-dinaphtho[2,3-a:2',3'-c]carbazol-6,11,12,17-tetron.

3. Verfahren zur Herstellung der Verbindungen der Formel (I) gemäß Anspruch 1,
dadurch **gekennzeichnet**, daß
es darauf beruht, daß man in einem Milieu aus Eisessig oder aus wässriger Essigsäure bei einer Temperatur von Umgebungstemperatur bis zu derjenigen des Rückfluß der Mischung ein 1,4-Naphthochinon der Formel (II) : 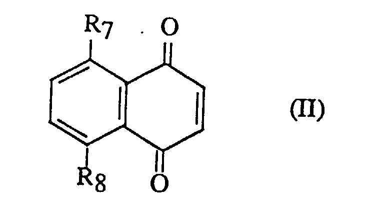 worin R₇ und R₈, unabhängig voneinander, ein Wasserstoffatom oder den Methoxyrest darstellen, mit einer Indolverbindung der Formel (III) reagieren läßt: 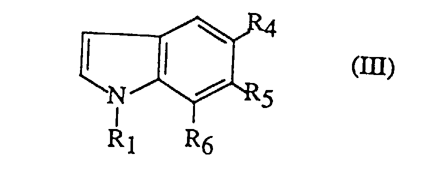 worin gilt:
R₁ stellt ein Wasserstoffatom, einen Benzyl- oder C₁₋₁₆-Alkylrest, verzweigt oder nicht, gesättigt oder nicht, dar;
R₄ stellt ein Wasserstoffatom, den Methoxy-, Nitril- oder Nitrorest oder ein Chloratom dar;
R₅ bedeutet ein Wasserstoffatom oder den Methoxyrest;
R₆ bedeutet ein Wasserstoffatom oder den Methylrest;
und daß man im selben Milieu die so erhaltene Indolyl-1,4-naphthochinonverbindung der Formel (IV) : 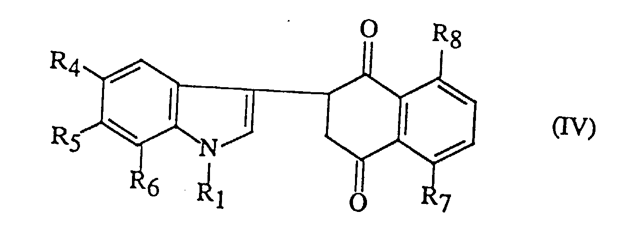 worin R₁, R₄, R₆, R₇ und R₈ die oben angegebenen Bedeutungen haben, mit einem Dienophil reagieren läßt, ausgewählt aus denen der folgenden Formeln: 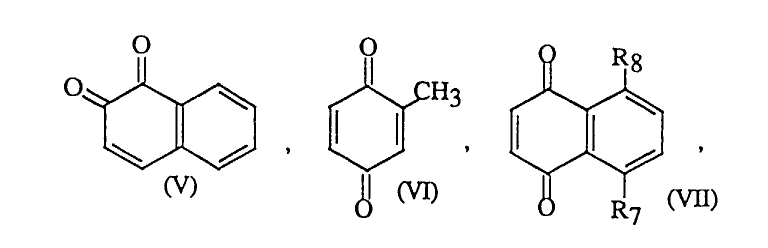 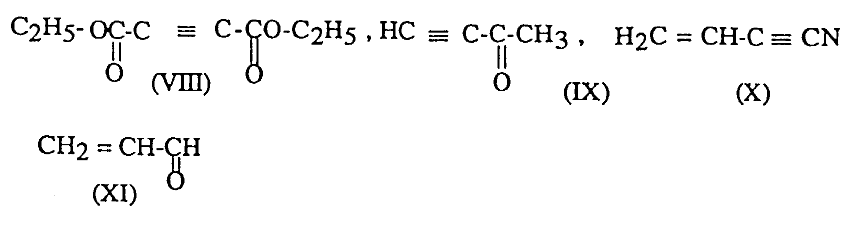 worin R₇ und R₈ die oben angegebenen Bedeutungen haben,
mit der Maßgabe, daß, wenn man das Indolyl-1,4-naphthochinon der Formel (IV) mit dem Dienophil der Formel (VII) reagieren läßt, R₁, R₄, R₅, R₆, R₇ und R₈ nicht gleichzeitig ein Wasserstoffatom bedeuten.

4. Verwendung der Verbindung der in Anspruch 1 definierten Formel (I) als Pigment in kosmetischen Zusammensetzungen.

5. Verwendung gemäß Anspruch 4,
dadurch **gekennzeichnet**, daß
die kosmetischen Zusammensetzungen Zusammensetzungen von Teint-Grundlagenprodukten, gefärbten Creme-Produkten, Wimperntuschen, Wangenschminkprodukten oder Lidschatten, Lippenstiften oder von Nagellacken und Zusammensetzungen zur zeitlichen Färbung der Haare darstellen.

6. Kosmetische Zusammensetzung zur kosmetischen Behandlung oder Färbung keratinischer Materien,
dadurch **gekennzeichnet**, daß
sie in einem kosmetisch geeigneten Milieu mindestens ein Pigment aus der Verbindung der in Anspruch 1 definierten Formel (I) enthält.

7. Zusammensetzung gemäß Anspruch 6,
dadurch **gekennzeichnet**, daß
das Pigment der Formel (I) in einer Konzentration von 0,1 bis 80 und vorzugsweise von 1 bis 30 Gew.% vorhanden ist, bezogen auf das Gesamtgewicht der Zusammensetzung.

8. Zusammensetzung gemäß einem der Ansprüche 6 und 7,
dadurch **gekennzeichnet**, daß
die Zusammensetzung in Form einer Lotion, verdickten Lotion, eines Gel, einer Emulsion, einer bläschenartigen Dispersion, Pulvers, Stifts vorliegt oder ggf. als Aerosol in Form eines Spray oder Schaums zubereitet ist.

9. Zusammensetzung gemäß einem der Ansprüche 6 bis 8,
dadurch **gekennzeichnet**, daß
sie Fettkörper, organische Lösungsmittel, Silicone, Verdickungsmittel, Weichmacher, oberflächenaktive Mittel, Sonnenfilterstoffe, Mittel gegen freie Radikale, Antischaummittel, Hydratisiermittel, Parfüm-Produkte, Konservierungsstoffe, Antioxidantien, Beladungsmittel, Sequestriermittel, Behandlungsmittel, Treibmittel, alkalisch oder sauer stellende Mittel oder weitere Pigmente enthält.

10. Zusammensetzung gemäß Anspruch 6,
dadurch **gekennzeichnet**, daß
sie dazu bestimmt ist, zur Färbung der Nägel verwendet zu werden und in Form einer Dispersion des Pigments in einem kosmetisch geeigneten Lösungsmittel vorliegt, das ein oder mehrere Harze enthält.

11. Zusammensetzung gemäß Anspruch 6,
dadurch **gekennzeichnet**, daß
sie dazu bestimmt ist, zur zeitlichen Färbung der Haare verwendet zu werden und in Form einer Dispersion des Pigments in einem zur Färbung geeigneten Milieu vorliegt.

12. Verfahren zum Schminken der Haut, der Wimpern oder der Augenbrauen,
dadurch **gekennzeichnet**, daß
man auf die Haut, die Wimpern oder die Augenbrauen mindestens eine in jedem der Ansprüche 6 bis 9 definierte Zusammensetzung aufbringt, die als Pigment mindestens ein Pigment der in Anspruch 1 oder 2 definierten Formel (I) enthält.

13. Verfahren zur Färbung der Nägel,
dadurch **gekennzeichnet**, daß
man auf die Nägel mindestens eine Zusammensetzung aufbringt, die in einem kosmetisch geeigneten Lösungsmittel ein oder mehrere Harze und mindestens ein Pigment aus der Verbindung der in den Ansprüchen 1 oder 2 definierten Formel (I) enthält.

14. Verfahren zur Färbung keratinischer Fasern, insbesondere der Haare,
dadurch **gekennzeichnet**, daß
man auf die Fasern mindestens eine Zusammensetzung aufbringt, die in einem zur Färbung geeigneten Milieu mindestens ein Pigment aus der Verbindung der in den Ansprüchen 1 oder 2 definierten Formel (I) enthält.

## Claims

1. Compound corresponding to the formula: 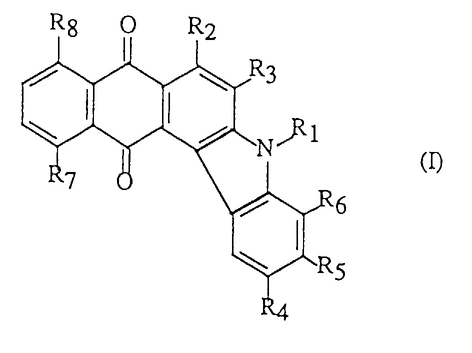 in which,
R₁ represents a hydrogen atom, a benzyl radical, or a C₁-C₁₆ alkyl radical which may or may not be branched and may or may not be saturated;
R₂ and R₃ denote, independently of each other, the carbethoxy, acetyl, nitrile or 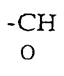 [sic] radical, or a hydrogen atom,
it not being possible, however, for R₂ and R₃ simultaneously to represent a hydrogen atom, or R₂ and R₃ form the following ring systems: 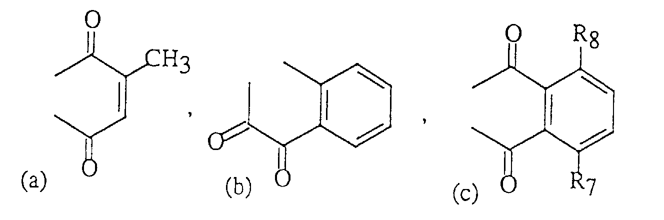
R₄ represents a hydrogen atom, the methoxy, nitrile or nitro radical or a chlorine atom;
R₅, R₇ and R₈ represent, independently of each other, a hydrogen atom or the methoxy radical;
R₆ represents a hydrogen atom or the methyl radical; with the proviso that if R₂ and R₃ form the ring system (c), at least one of R₁, R₄, R₅, R₆, R₇ and R₈ does not denote a hydrogen atom.

2. Composition according to Claim 1, characterized in that it is chosen from
- 2,3-dimethoxy-5-H-dinaphtho[2,3-a:2',3'-c]-carbazole-6,11,12,17-tetrone,
- 10,13-dimethoxy-5H-dinaphtho[2,3-a:2',3'-c]-carbazole-6,11,12,17-tetrone,
- 7,16-dimethoxy-5H-dinaphtho[2,3-a:2',3'-c]-carbazole-6,11,12,17-tetrone,
- 7,13-dimethoxy-5H-dinaphtho[2,3-a:2',3'-c]-carbazole-6,11,12,17-tetrone,
- 10,16-dimethoxy-5H-dinaphtho[2,3-a:2',3'-c]-carbazole-6,11,12,17-tetrone,
- 2-methoxy-5H-dinaphtho[2,3-a:2',3'-c]carbazole-6,11,12,17-tetrone,
- 4-methyl-5H-dinaphtho[2,3-a:2',3'-c]carbazole-6,11,12,17-tetrone,
- 2-chloro-5H-dinaphtho[2,3-a:2',3'-c]carbazole-6,11,12,17-tetrone,
- 7-methyl-5-hexadecylnaphtho[2,3-a]benzo[2',3'-c]-carbazole-6,9,10,15-tetrone,
- 5-hexadecyldinaphtho[2,3-a:2',3'-c]carbazole-6,11,12,17-tetrone,
- 2-cyano-5H-dinaphtho[2,3-a:2',3'-c]carbazole-6,11,12,17-tetrone,
- 2-nitro-10,13-dimethoxy-5H-dinaphtho-[2,3-a:2',3'-c]carbazole-6,11,12,17-tetrone,
- 2-nitro-7,16-dimethoxy-5H-dinaphtho-[2,3-a:2',3'-c]carbazole-6,11,12,17-tetrone,
- 2-nitro-7,13-dimethoxy-5H-dinaphtho-[2,3-a:2',3'-c]carbazole-6,11,12,17-tetrone,
- 2-nitro-10,16-dimethoxy-5H-dinaphtho-[2,3-a:2',3'-c]carbazole-6,11,12,17-tetrone.

3. Process for the preparation of the compounds of formula (I) according to Claim 1, characterized in that it consists in reacting, in a medium consisting of glacial acetic acid or of aqueous acetic acid and at a temperature ranging from room temperature to the reflux temperature of the mixture, a 1,4-naphthoquinone of formula (II) 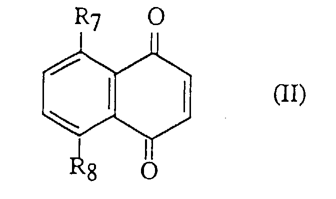 in which R₇ and R₈ represent, independently of each other, either a hydrogen atom or the methoxy radical, with an indole compound of formula (III) 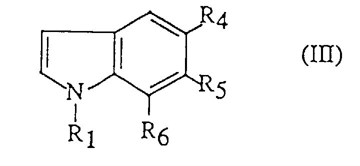 in which
R₁ represents a hydrogen atom, a benzyl radical or a C₁-C₁₆ alkyl radical which may or not be branched and may or may not be saturated;
R₄ represents a hydrogen atom, the nitrile, methoxy or nitro radical or a chlorine atom;
R₅ denotes a hydrogen atom or the methoxy radical,
R₆ denotes a hydrogen atom or the methyl radical,
in reacting in a same medium the indolyl-1,4-naphthoquinone compound of formula (IV) thus obtained: 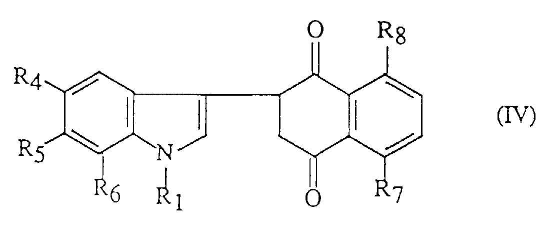 in which R₁, R₄, R₅, R₆, R₇ and R₈ have the same meanings as indicated above, with a dienophile chosen from those corresponding to the following formulae: 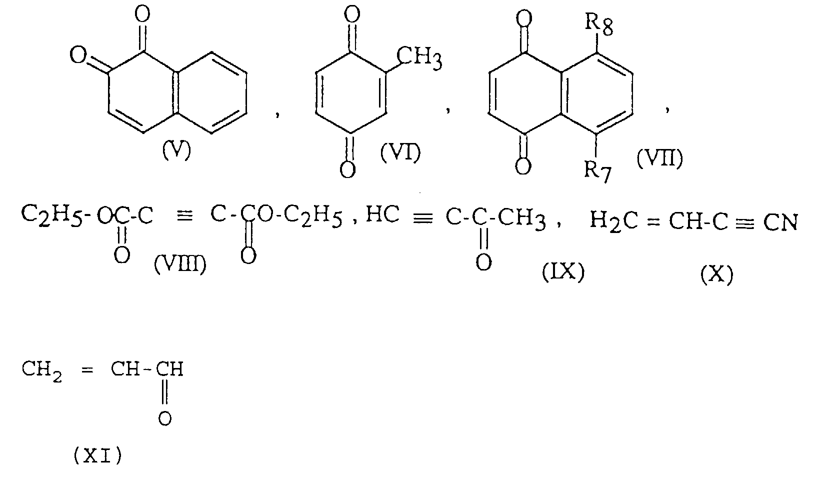 with R₇ and R₈ having the same meanings as indicated above;
with the proviso that if the indolyl-1,4-naphthoquinone of formula (IV) is reacted with the dienophile of formula (VII), R₁, R₄, R₅, R₆, R₇ and R₈ cannot simultaneously denote a hydrogen atom.

4. Use as a pigment in cosmetic compositions of the compound corresponding to the formula (I) as defined in Claim 1.

5. Use according to Claim 4, characterized in that the cosmetic compositions consist of foundation compositions, tinted creams, mascaras, blushers or eye shadows, lipsticks, nail varnishes and nonpermanent dyeing compositions for the hair.

6. Cosmetic composition intended for the cosmetic treatment or for the coloration of keratinous substances, characterized in that it contains, in a cosmetically acceptable medium, at least one pigment consisting of the compound corresponding to the formula (I) as defined in Claim 1.

7. Composition according to Claim 6, characterized in that the pigment of formula (I) is present at a concentration of between 0.1 and 80%, and preferably of between 1 and 30%, by weight relative to the total weight of the composition.

8. Composition according to either of Claims 6 and 7, characterized in that the composition takes the form of a lotion, a thickened lotion, a gel, an emulsion, a vesicle dispersion, a powder or a stick, or is optionally packaged as an aerosol in the form of a spray or a foam.

9. Composition according to any one of Claims 6 to 8, characterized in that it contains fatty substances, organic solvents, silicones, thickening agents, softening agents, surfactants, sunscreen agents, anti-free-radical agents, anti-foaming agents, moisturizing agents, fragrances, preserving agents, antioxidants, fillers, sequestering agents, treatment agents, propellants, basifying agents or acidifying agents, or other pigments.

10. Composition according to Claim 6, characterized in that it is intended to be used for colouring the nails and in that it takes the form of a dispersion of the pigment in a cosmetically acceptable solvent containing one or more resins.

11. Composition according to Claim 6, characterized in that it is intended to be used for the nonpermanent dyeing of hair and in that it takes the form of a dispersion of the pigment in a medium which is suitable for dyeing.

12. Process for making up the skin, the eyelashes or the eyebrows, characterized in that at least one composition as defined in any one of Claims 6 to 9 and containing as pigment at least one pigment of formula (I) as defined in Claims 1 or 2 is applied to the skin, the eyelashes or the eyebrows.

13. Process for colouring the nails, characterized in that at least one composition containing, in a cosmetically acceptable solvent, one or more resins and at least one pigment consisting of the compound of formula (I) as defined in Claims 1 or 2 is applied to the nails.

14. Process for dyeing keratinous fibres, in particular the hair, characterized in that at least one composition containing, in a medium which is suitable for dyeing, at least one pigment consisting of the compound of formula (I) as defined in Claims 1 or 2 is applied to the fibres.
